# EUROPEAN PATENT APPLICATION

(11) **EP 1 408 332 A1**
(43) Date of publication of application: **14.04.2004**
(21) Application number: 03466017.5
(22) Date of filing: 07.10.2003
(51) Int. Cl.: G01N 33/36, B65H 63/06, G01B 11/10, G01N 21/89

(54) **A device for monitoring a moving linear textile formation, in particular a yarn**

(30) Priority: 08.10.2002 CZ 20023337
(71) Applicant: Rieter CZ a.s., 562 15 Usti nad Orlici (CZ)
(72) Inventor: Stusak, Miroslav, V zahradach 458 (CZ); Sloupensky, Jiri, Cs armady 869 (CZ)
(74) Representative: Musil, Dobroslav, Dipl.-Ing.

(57) **Abstract**

The invention relates to a device for monitoring a moving linear textile formation, in particular a yarn, comprising an operating means (3) with at least one row of radiation-sensitive elements and means for coupling the operating means (3) with circuits processing and evaluating parameters of the monitored moving linear textile formation where the operating means (3) is positioned in a case whose front panel (1) is transparent at least in the area of radiation-sensitive elements of the operating means (3) and is composed of a part of a moving linear textile formation guide.

## Description

### Technical field

The invention relates to a device for monitoring a moving linear textile formation, in particular a yam, comprising an operating means with at least one row of radiation-sensitive elements and means for coupling the operating means with circuits processing and evaluating parameters of the monitored moving linear textile formation where the operating means is positioned in a case whose front panel is transparent at least in the area of radiation-sensitive elements of the operating means.

### Background art

Optical sensors produced at present consist of an operating means with a plurality of radiation-sensitive elements and, as the case may be, also with support circuits and processors while the operating means must be incased in appropriate manner to permit manipulation with the optical sensor while producing various optical devices employing mentioned sensors. The operating means with a system of radiation-sensitive elements is, including eventual support circuits and processors, by a suitable technology for integrated circuit production produced on an appropriate vehicle which is as a rule in the form of a plate. The sensing side of the operating means is situated on the side opposite to the vehicle while on the sensing side of the operating means at least in the area of radiation-sensitive elements a transparent front panel of the case made for instance of a glass plate is positioned. The height differences of particular parts of the operating means of the optical sensor in regard to the vehicle level on which the operating means is positioned are leveled up by applying a special optically pure gel between the vehicle with the operating means and the front panel of the case. Such an incased optical sensor can be used in various applications.

CZ PV 2001-121 discloses a device for monitoring a moving linear textile formation, in particular a yarn, comprising the operating means with a plurality sensing elements arranged next to each other. The operating means is incased by above mentioned manner and along with the case forms the entire sensor while it is by the front panel transparent at least in the area of radiation-sensitive elements of the operating means bonded to one side of the operating means' front panel transparent at least in the area of radiation-sensitive elements where the panel itself has textile-advantageous features, for instance abrasion resistance (hardness) and which is a component of a yarn guide comprising also a second transparent panel parallel to the sensor panel. The transparent panel of the yarn guide is at least in the area of the radiation-sensitive elements of the operating means bonded onto the front panel of the sensor case and is being made for example of glass. Between the sensor and the transparent panel of the yarn guide or onto the transparent panel surface can be an optical screen bonded. The sensor itself can be composed of CCD optical sensor or CMOS optical sensor.

The production of this device in spite of its advantageous end-use properties discloses also a disadvantage that the device consists of relatively high number of mutually connected parts requiring higher demands in particular on accuracy and labour consumption and these facts negatively affect in particular the price of the device. Another disadvantage of the device is the presence of the bonded joint at least in the area of the radiation-sensitive elements of the sensor case transparent front panel and the bonded joint of the transparent panel of the yarn guide where the glue layer can affect negatively radiation transmission to the effective elements (radiation-sensitive elements) of the sensor (the operating means) which can affect negatively the accuracy of monitoring the moving linear textile formation or, as the case may be, the changes in optical properties of the system due to ageing processes of the glue can come into effect etc.

The goal of the invention intends to eliminate or at least to minimize the drawbacks of the art.

### Principle of the invention

The goal of the invention has been reached by a device for monitoring the moving linear textile formation, in particular a yarn, whose principle consists in that at least in the area of the radiation-sensitive elements of the operating means the transparent front panel of the operating means case is composed of a part of the moving linear textile formation guide. A highly integrated device is produced by this manner comprising radiation-transmitting part of the moving linear textile formation guide so it can be easily integrated into a particular place or into a particular functional complex. Another advantage of this embodiment consists in that there is no auxiliary means for connecting the moving linear textile formation guide with the sensing element employed so all possible negative effects in imprecision of mounting and possible effects of the glue layer are prevented etc.

According to a few preferred embodiments, for instance as shown in the Fig. 1 and 2 or according to embodiment of the U-shaped guide shown in the Fig. 4 etc. the front panel of the operating means case consists of one of the two parallel, in distance situated flat parts of the moving linear textile formation guide which are connected together on one end of the guide.

For a larger scale of the device integration it is preferred when on the part of the moving linear textile formation guide opposite to the front panel of the operating means case a radiation source is positioned.

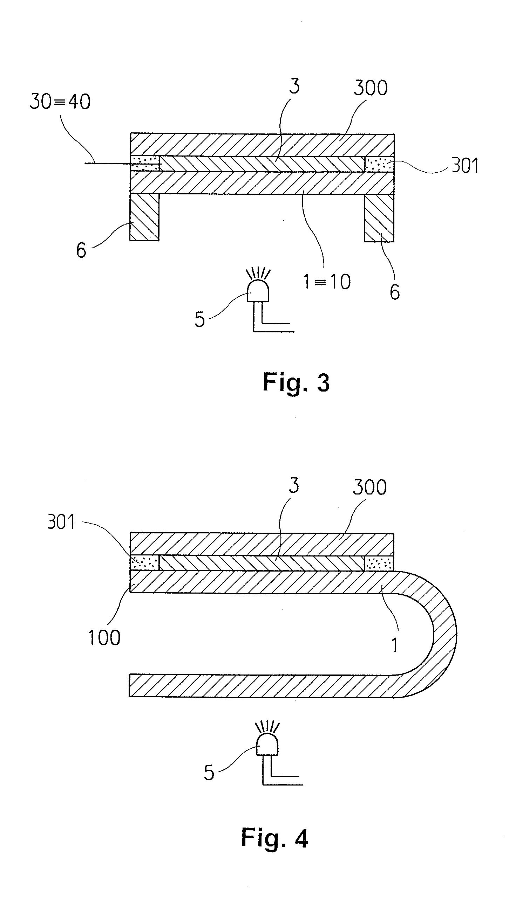

According to another preferred embodiment of the guide, see Fig. 3, the front panel of the operating means case is composed of a flat panel which is on the front side on both ends fitted with a lug.

To improve the optical properties of the device it is preferred when the front panel of the operating means case is composed of the optical element with appropriate optical properties, e.g. radiation alignment etc. For instance for reduction of the background radiation effects it is preferred when at least on the front panel of the operating means case is a non-transparent optical screen positioned and in the place of radiation-sensitive elements of the operating means fitted with longitudinal slot.

From the view of materials it is preferred when the front panel of the operating means case consists of a transparent glass panel or a clear plastic panel. Front panel of the operating means case can also consist of a panel made of glass or a plastic material transmitting only the radiation in a wavelength transmitted from the radiation source and, as the case may be, transmitting the radiation in wavelengths from the range in the vicinity of the wavelength transmitted from the radiation source which easily forms an undesired wavelengths filter, filtering for instance the backlight wavelengths radiation which lowers the effect of the backlight on the device function.

According to one embodiment the operating means is integrated in one case and/or advantageously on one substrate panel together with a semiconductor customer ASIC comprising at least a part of electronic circuits for processing and/or evaluating the signals of operating means and is fitted with outlets for connecting to superior evaluating and/or control system. This embodiment is characterized by a high degree of integration with other devices of the entire moving linear textile formation monitoring system while maintaining all the advantages of the case front panel being a part of the moving linear textile formation guide. The operating means can also be an integral part of the semiconductor customer ASIC which represents next, higher level of integration.

The operating means can consist of CMOS optical sensing element which is a technology preferred for integration of sequential electronic circuits for processing and/or evaluating the signal from the operating mean.

### Description of the drawing

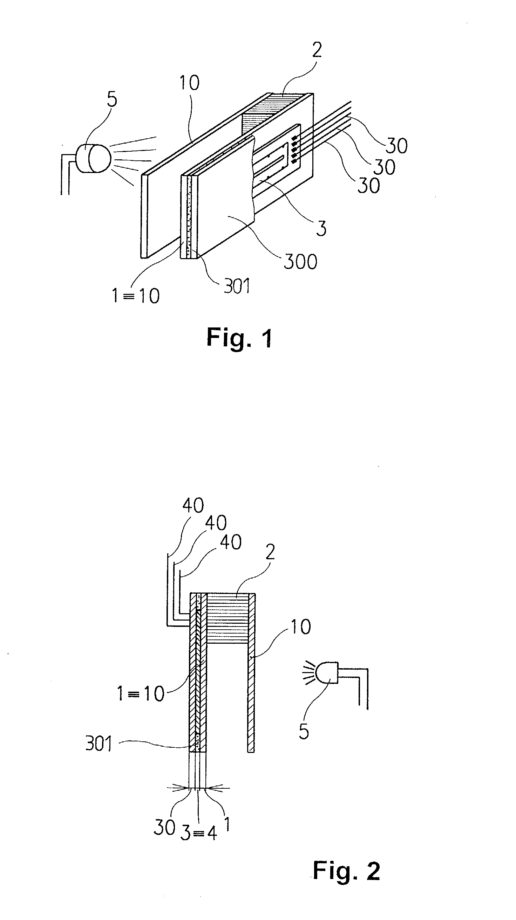

The invention is schematically shown in the drawings in which Fig. 1 is the floor projection of a device for monitoring the moving linear textile formation, in particular a yarn, with at least a part of a linear textile formation guide, Fig. 2 is the perspective view of a device for monitoring the moving linear textile formation, in particular a yarn, integrated into the linear textile formation guide from the Fig. 1, Fig. 3 is another example of embodiment of a device for monitoring the moving linear textile formation, in particular a yarn, and Fig. 4 is another example of embodiment of a device for monitoring the moving linear textile formation, in particular a yarn.

### Specific description

Device for monitoring a moving linear textile formation, in particular a yarn, consisting of an operating means **3** for a moving linear textile formation monitoring positioned in a case which is at least from the side of radiation-sensitive elements at least in a part of the radiation-sensitive elements area of the operating means 3 transparent for at least the wavelength transmitted by a radiation source **5**. Thus the device forms one body and is intended for use as a sensing element in optical system sensing parameters of the moving linear textile formation. The device can be connected to next elements and a device of the entire system evaluating parameters of the moving linear textile formation.

The operating means **3** consists on its front panel of at least one row of radiation-sensitive elements (pixels) and is composed of CMOS optical sensing element, or as the case may be of CCD optical sensing element and thus the resulting output is a digital signal. The operating means **3** further consists of means for coupling with circuits necessary for processing and evaluating parameters of the monitored moving linear textile formation as described for instance in CZ PV 2001-4561. In the embodiment according to Fig. 1 is the operating means **3** fitted with outlets **30** for connecting to next circuits necessary for processing and evaluating signals of the operating means **3**.

The operating means **3** can also be a part of a semiconductor customer ASIC **4** comprising at least a part of electronic circuits for processing and/or evaluation the signals of operating means **3**. Thus the operating means **3** can for instance be made by the similar technology with semiconductor customer ASIC **4** on a shared substrate panel forming one integrated circuit, e.g. CMOS integrated circuit with CMOS optical sensing element. Semiconductor customer ASIC **4** thus processes and evaluates at least a part of parameters of the monitored moving linear textile formation and is fitted with outlets **40** (see Fig. 2) for connecting to superior evaluating and/or control device, for instance an operating means control device or a section of operating means or the entire machine etc.

The operating means **3** is positioned in a case in the manner that the operating means **3**, or as the case may be the entire semiconductor customer ASIC 4 which the operating means **3** can be a part of, etc., is by its back panel, i.e. the panel without radiation-sensitive elements using an appropriate technology of electronic components production and/or circuits, e.g. vapour deposition technology, made on the front panel of an appropriate vehicle **300** which can serve as the back panel of the operating means case **3** or which is positioned on the back panel of the operating means **3** case. The operating means **3** can be on the vehicle **300** made including eventual support connections and circuits. Also eventual semiconductor customer ASIC **4** comprising operating means **3** can be by its back panel, that is by the side without radiation-sensitive elements of the operating means **3** made on the vehicle **300**.

The front panel **1** of the case is at least in the area of radiation-sensitive elements of the operating means **3** transparent at least for the wavelength transmitted by the radiation source **5** and covers the front panel of the operating means **3** and the rest surface of the vehicle **300** front panel, i.e. the vehicle **300** front panel surface not covered by the operating means **3** and by eventual next circuits. Between the case front panel **1** and the vehicle **300** with the operating means **3** is a not represented special optically pure gel **301** to level up unevenness caused by forming the operating means **3** and eventual next circuits and connections on the vehicle **300** front panel. The special optically pure gel **301** serves not only to fill the empty space between the case front panel **1** and the vehicle **300** with the operating means **3** (air or industrial vacuum are less preferred) but also serves to fix particular parts of the device, e.g. outlets from the operating means **3** or eventual next circuits or connections etc.

The front panel **1** of the operating means **3** case consists of the moving linear textile formation guide and is made of textile- and optically appropriate material, e.g. it is made of a special glass for use in the textile industry or an appropriate plastic material etc. It is important so that the material used for production of the front panel **1** of the operating means **3** case is sufficiently abrasion resistant to the moving linear textile formation and also has sufficient optical properties for the transmission of the source **5** radiation onto the radiation-sensitive elements of the operating means **3** behind the case front panel **1.** At the same time this material serves as a filter of an undesirable backlight radiation (light) while transmitting only the radiation transmitted from the radiation source **5**, or as the case may be transmitting the radiation in wavelengths from the range in the vicinity of the wavelength transmitted from the radiation source **5.**

In the example of embodiment shown in Fig. 1 and 2 the case front panel **1** forms one part **10** of the guide channel of the moving linear textile formation guide. Moving linear textile formation guide consists of a pair of in distance situated and preferably parallel parts **10** between which is from one side of the guide positioned a gap for entering and moving the moving linear textile formation between the guide parts **10**. The guide parts **10** are by its second end positioned, e.g. by bonding etc., on the separating body **2** which can for example be a cross wall and/or a transparent body according to CZ PV 2001-121. Both parallel parts **10** of the guide in the example of embodiment shown in Fig. 1 and 2 consist of flat clear glass plates for use in the textile industry or an appropriate clear plastic material etc. The separating body **2** preferably has the same properties as both parallel parts **10** of the guide. In the example of embodiment shown in Fig. 3 the case front panel **1** is composed of a flat panel which is on its outer side fitted with one lug **6** on each end while the moving linear textile formation guide is composed of the case front panel **1** and a pair of in distance situated lugs **6**.

In the example of embodiment shown in Fig. 4 the case front panel **1** is an integral part of the U-shaped moving linear textile formation guide where it forms one arm of the U-shaped moving linear textile formation guide.

In the not represented example of embodiment is the case front panel **1** a part of suitably constructed moving linear textile formation guide.

On the case front panel **1** can in any of above mentioned embodiments formed a non transparent optical screen fitted with a slot only in the area of radiation-sensitive elements which enables the transmission of the radiation from the radiation source **5** onto the radiation-sensitive elements of the operating means **3** and prevents the incidence from other radiation sources and from other directions onto the radiation-sensitive elements of the operating means **3**. The case front panel **1** can form an auxiliary optical element. Also other parts of the moving linear textile formation guide can be fitted with a screen with a longitudinal slot, for instance also both parallel parts **10** of the guide from examples of embodiment according to Fig. 1, 2 and 4 so that the radiation (light) from the radiation source **5** situated behind the second part **10** could reach the radiation-sensitive elements of the operating means **3** behind the first part **10** of the guide (case front panel **1).**

A part of the operating means **3** can consist of at least one reference radiation-sensitive element which is for example according to Fig. 1 and 2 situated behind the separating body **2.**

Particular parts forming the moving linear textile formation guide, for instance both guide parts **10** and respectively also the separating body **2** from the embodiment according to Fig. 1 and 2 can be on its surface situated in the vicinity of the area for passing the monitored moving linear textile formation fitted with a suitable abrasion resistance coat for enhancing its abrasion resistance.

In the example of embodiment shown in Fig. 1 and 2 can the separating body **2** of the guide be at least in the part of its surface in the vicinity of the area for passing the moving linear textile formation between both parallel parts **10** of the guide fitted with a radiation opaque coat forming the well-known cross wall.

The above mentioned radiation source **5** can figure in the system of monitoring the parameters of the moving linear textile formation mounted in position appropriate to the operating means **3**, e.g. in embodiment according to Fig. 1, 2 and 4 it can be positioned directly in the second part **10** of the guide opposite the case front panel **1**. respectively in the second arm of the U-shaped guide or it can be mounted on the outer surface of the second part **10** of the guide, respectively on the second U-shaped arm of the guide while also this second part **10** of the guide, respectively the second U-shaped arm of the guide can form the support optical element or between the second part **10** of the guide, respectively the second U-shaped arm of the guide and the radiation source **5** there can be a fixation optical element etc.

### lndustrial applicability

The invention can be applied on textile machines for monitoring a yarn or another linear textile material.

## Claims

1. A device for monitoring a moving linear textile formation, in particular a yarn, comprising an operating means with at least one row of radiation-sensitive elements and means for coupling the operating means with circuits processing and evaluating parameters of the monitored moving linear textile formation where the operating means is positioned in a case whose front panel is transparent at least in a part of the area of radiation-sensitive elements of the operating means, **characterized by** that the case front panel (1) of the operating means (3) case is composed of a part of the moving linear textile formation guide.

2. A device as claimed in Claim 1, **characterized by** that the front panel (1) of the operating means (3) case consists of one of the two parallel flat parts (10) of the moving linear textile formation guide which are connected together on one end of the guide.

3. A device as claimed in any of the Claims 1 or 2, **characterized by** that on a part of the moving linear textile formation guide opposite to the front panel (1) of the operating means (3) case a radiation source (5) is positioned.

4. A device as claimed in Claim 1, **characterized by** that the front panel (1) of the operating means (3) case is composed of a flat panel which is on the front side on both ends fitted with a lug.

5. A device as claimed in any of Claims 1 to 4, **characterized by** that the front panel (1) of the operating means (3) case is composed of an optical element.

6. A device as claimed in any of Claims 1 to 5, **characterized by** that at least on the front panel (1) of the operating means (3) case is a non transparent optical screen positioned while it is in the area of radiation-sensitive elements of the operating means (3) fitted with a longitudinal slot.

7. A device as claimed in any of Claims 1 to 6, **characterized by** that the front panel (1) of the operating means (3) case is composed of a transparent glass panel.

8. A device as claimed in any of Claims 1 to 7, **characterized by** that the front panel (1) of the operating means (3) case is composed of a panel made of a transparent plastic material.

9. A device as claimed in any of Claims 1 to 6, **characterized by** that the front panel (1) of the operating means (3) case is composed of a transparent glass panel transmitting only the radiation in a wavelength transmitted from the radiation source (5) and, as the case may be transmitting also the radiation in wavelengths from the range in the vicinity of the wavelength transmitted from the radiation source (5).

10. A device as claimed in any of Claims 1 to 6, **characterized by** that the front panel (1) of the operating means (3) case is composed of a transparent plastic material panel transmitting only the radiation in the wavelength transmitted from the radiation source (5) and, as the case may be transmitting also the radiation in wavelengths from the range in the vicinity of the wavelength transmitted from the radiation source (5).

11. A device as claimed in any of Claims 1 to 10, **characterized by** that the operating means (3) is positioned in the case along with a semiconductor customer ASIC (4) comprising at least a part of electronic circuits for processing and/or evaluating signals of the operating means (3) and is fitted with outlets (40) for connecting to superior evaluating and/or control means.

12. A device as claimed in Claim 11, **characterized by** that the operating means (3) is an integral part of the semiconductor customer ASIC (4).

13. A device as claimed in any of Claims 1 to 12, **characterized by** that the operating means (3) is composed of a CMOS optical sensing element.
